# EUROPEAN PATENT APPLICATION

(11) **EP 4 268 756 A1**
(43) Date of publication of application: **01.11.2023**
(21) Application number: 21908966.1
(22) Date of filing: 18.11.2021
(51) Int. Cl.: A61B 34/35, A61B 17/00, A61B 90/00, H04N 7/18

(54) **REMOTE SURGERY SYSTEM AND CONTROL METHOD BASED ON LAPAROSCOPIC SURGERY ROBOT**

(30) Priority: 24.12.2020 CN 202011549192
(71) Applicant: Harbin Intelligent Surgery Equipment Co., Ltd., Harbin, Heilongjiang 150000 (CN)
(72) Inventor: ZHAN, Mengxu, Harbin, Heilongjiang 150000 (CN); PANG, Haifeng, Harbin, Heilongjiang 150000 (CN)
(74) Representative: Zaboliene, Reda
(86) International application number: PCT/CN2021/131420
(87) International publication number: WO 2022/134967

(57) **Abstract**

Provided are a remote surgery system and control method based on a laparoscopic surgery robot, relating to the technical field of surgery robots. The remote surgery system based on the laparoscopic surgery robot comprises a laparoscopic surgery robot system and a remote communication system, wherein the laparoscopic surgery robot system comprises a surgical robot arm located on a patient side and a console located on a doctor side; and the surgical robot arm and the console are suitable for communication connection by means of the remote communication system. Remote surgery on a patient by a doctor is enabled by means of the remote communication system, and the surgery can be performed when the patient side and the doctor side are not in a same physical space, thereby enabling wide-spread access to high-quality medical resources; since control instructions are subjected to data calculation by using an algorithm only at the surgical robot arm, the time delay of devices occupied by the console and the surgical robot arm calculating data separately is reduced, thereby ensuring real-time performance of surgical operations.

## Description

### Technical Field

The present invention relates to the technical field of surgery robots, and more particularly to a remote surgery system and control method based on a laparoscopic surgery robot.

### Background Art

With the development of laparoscopic technology and surgery robots, an application range of the laparoscopic surgery robots is enlarged continuously. The laparoscopic surgery robots can partially replace boring, repetitive and tiring operations; and the difficult operations such as anastomoses of small pipes during the traditional laparoscopic surgery can be completed by using the image stability of a robot system and exquisite instruments. The cooperation between doctors and the robots can improve the surgery quality to certain extent. At present, for the minimally invasive surgery based on the laparoscopic robot, patients and the doctors are at the same site, which is a prerequisite condition for performing the surgery; this condition disables a lot of patients in remote areas and special circumstances to obtain best treatment opportunities due to the inconvenient transportations; and therefore, how to provide wide-spread high-quality medical services becomes a key point of the medical upgrading at present.

### Summary of the Invention

The problem to be solved by the present invention is how to provide wide-spread high-quality medical services.

In order to solve the above problems, the present invention provides a remote surgery system based on a laparoscopic surgery robot, which includes a laparoscopic surgery robot system and a remote communication system, wherein the laparoscopic surgery robot system includes a surgical robot arm located on a patient side and a console located on a doctor side; the surgical robot arm and the console are suitable for communication connection by means of the remote communication system; the surgical robot arm is used for receiving a control instruction transmitted by the console through the remote communication system, parsing the control instruction so as to determine an end pose of the surgical robot arm, and transmitting own status information through the remote communication system; and the console is used for generating the control instruction, transmitting the control instruction through the remote communication system, and receiving the status information transmitted by the surgical robot arm through the remote communication system.

According to the remote surgery system based on the laparoscopic surgery robot of the present invention, remote surgery on a patient by a doctor is enabled by means of the remote communication system, so that the surgery can be performed when the patient side and the doctor side are not in a same physical space, thereby enabling wide-spread access to high-quality medical resources; and since the control instructions are subjected to the data calculation by using an algorithm only at the surgical robot arm, the time delay of devices occupied by the console and the surgical robot arm calculating data separately is reduced, thereby ensuring the real-time performance of surgical operations.

Optionally, the remote surgery system based on the laparoscopic surgery robot also includes a 3D intra-cavity video processing system and a 3D display located on the doctor side; the 3D intra-cavity video processing system includes a laparoscopic lens, an intra-cavity image collection unit and a video encoding/decoding system; the laparoscopic lens is installed on the surgical robot arm; the laparoscopic lens is used for collecting focus information of the patient; the intra-cavity image collection unit is used for receiving the focus information collected by the laparoscopic lens and transmitting the focus information to the video encoding/decoding system; and the video encoding/decoding system is used for encoding/decoding the focus information and transmitting to the 3D display through the remote communication system.

According to the remote surgery system based on the laparoscopic surgery robot of the present invention, the visualization of a surgical procedure is realized through the 3D intra-cavity video processing system, so that the doctor adjusts the surgical situation in time, thereby effectively improving the safety of the laparoscopic surgery.

Optionally, the video encoding/decoding system includes a video capture card, a video transmission device and a video receiving device; the video capture card is connected with the intra-cavity image collection unit; the video capture card is used for receiving the focus information transmitted by the intra-cavity image collection unit and transmitting the focus information to the video transmission device; the video transmission device is used for encoding the focus information and transmitting to the video receiving device through the remote communication system; and the video receiving device is used for receiving the focus information transmitted by the video transmission device, decoding the focus information and transmitting the decoded focus information to the 3D display, so that the focus information is displayed on the 3D display.

According to the remote surgery system based on the laparoscopic surgery robot of the present invention, the video encoding/decoding system encodes/decodes a 3D laparoscopic image containing the focus information to realize the visualization of the surgical procedure, so that the doctor adjusts the surgical situation in time, thereby effectively improving the safety of the laparoscopic surgery.

Optionally, the remote communication system includes a video transmission communication system, a video receiving communication system and a network base station; the video transmission communication system is connected with the video transmission device; the video transmission communication system is used for receiving the focus information transmitted by the video transmission device and transmitting the focus information to the video receiving communication system through the network base station; the network base station is communicated with the video transmission communication system and connected with the video receiving communication system; the network base station is used for receiving the focus information transmitted by the video transmission communication system and transmitting the focus information to the video receiving communication system; the video receiving communication system is connected with the 3D display; the video receiving communication system is used for receiving the focus information transmitted by the network base station and transmitting the focus information to the 3D display; and the video transmission communication system and the video receiving communication system are suitable for transmitting the focus information through a first dedicated circuit.

According to the remote surgery system based on the laparoscopic surgery robot of the present invention, through the remote communication system, the transmission of the 3D laparoscopic image containing the focus information is enabled, and the visualization of the surgical procedure is realized, so that the doctor adjusts the surgical situation in time, thereby effectively improving the safety of the laparoscopic surgery; and the same time, since the focus information is transmitted between the video transmission communication system and the video receiving communication system through the first dedicated circuit, which does not interfere with other data transmission, the mutual interference is reduced, the collateral damage caused by the network instability is prevented, and the reliability of the data transmission is ensured.

Optionally, the video transmission device is suitable for adopting a UDP protocol when transmitting the focus information to the video receiving device.

According to the remote surgery system based on the laparoscopic surgery robot of the present invention, through the UDP protocol, the focus information is transmitted by the video transmission device to the video receiving device, which ensures the high real-time performance of the data transmission, and gives the doctor good surgical experience, so that the doctor adjusts the surgical situation in time, thereby effectively improving the safety of the laparoscopic surgery.

Optionally, the video transmission device is suitable for adopting a low-distortion encoding algorithm when encoding the focus information.

According to the remote surgery system based on the laparoscopic surgery robot of the present invention, the focus information is encoded through the low-distortion encoding algorithm; when a network status is affected, a compression ratio of the 3D image video may be adjusted according to the network status to ensure the high continuity of the 3D image, so that the doctor adjusts the surgical situation in time, thereby effectively improving the safety of the laparoscopic surgery.

Optionally, the remote communication system also includes a robot arm communication system and a console communication system; the robot arm communication system is connected with the surgical robot arm; the console communication system is connected with the console; the robot arm communication system is communicated and connected with the console communication system; and the robot arm communication system and the console communication system are suitable for transmitting the control instructions and the status information through a second dedicated circuit.

According to the remote surgery system based on the laparoscopic surgery robot of the present invention, the data transmission between the surgical robot arm and the console communication system is enabled through the robot arm communication system and the console communication system to realize the remote surgery on the patient by the doctor, so that the surgery can be performed when the patient side and the doctor side are not in the same physical space, thereby enabling the wide-spread access to the high-quality medical resources; and at the same time, since the control instructions and the status information are transmitted between the robot arm communication system and the console communication system through the second dedicated circuit, which does not interfere with other data transmission, the mutual interference is reduced, the collateral damage caused by the network instability is prevented, and the reliability of the data transmission is ensured.

Optionally, the surgical robot arm determines the end pose based on a kinematics algorithm.

According to the remote surgery system based on the laparoscopic surgery robot of the present invention, the end pose of the surgical robot arm is determined through the kinematics algorithm, so that the remote surgery on the patient by the doctor is realized, and the surgery can be performed when the patient side and the doctor side are not in the same physical space, thereby enabling the wide-spread access to the high-quality medical resources.

Optionally, the surgical robot arm includes a surgical robot arm body and a surgical instrument apparatus for performing the surgery on the patient; and the surgical instrument apparatus is installed on the surgical robot arm body.

According to the remote surgery system based on the laparoscopic surgery robot of the present invention, through the surgical instrument apparatus installed on the surgical robot arm body, the remote surgery on the patient by the doctor is realized, so that the surgery can be performed when the patient side and the doctor side are not in the same physical space, thereby enabling the wide-spread access to the high-quality medical resources.

The present invention further provides a control method based on a laparoscopic surgery robot, which includes: a control instruction is generated and the control instruction is transmitted to a surgical robot arm through a remote communication system by a console; the control instruction is parsed by the surgical robot arm so as to determine an end pose of the surgical robot arm; and status information of the surgical robot arm is transmitted to the console by the surgical robot arm.

The control method based on the laparoscopic surgery robot has the same advantages as that of the remote surgery system based on the laparoscopic surgery robot compared with the prior art, which is not repeated here.

### Brief Description of the Drawings

FIG. 1 is a block diagram I of a remote surgery system based on a laparoscopic surgery robot in an embodiment of the present invention.
FIG. 2 is a block diagram II of the remote surgery system based on the laparoscopic surgery robot in an embodiment of the present invention.

### Detailed Description of the Invention

To make the above purposes, characteristics and advantages of the present invention more apparent and understood, specific embodiments of the present invention are described in detail below in combination with the accompanying drawings.

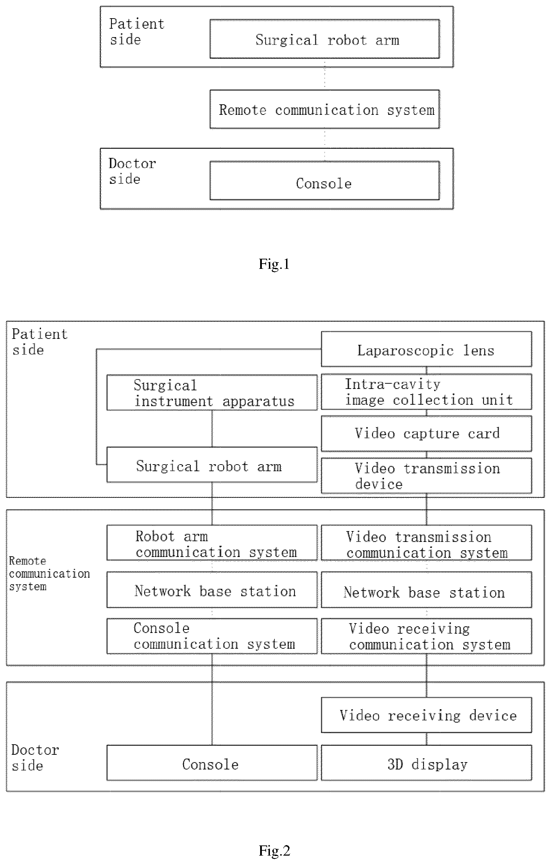

As shown in FIG. 1, an embodiment of the present invention provides a remote surgery system based on a laparoscopic surgery robot, which includes a laparoscopic surgery robot system and a remote communication system; the laparoscopic surgery robot system includes a surgical robot arm located on a patient side and a console located on a doctor side; the surgical robot arm and the console are suitable for communication connection by means of the remote communication system; the surgical robot arm is used for receiving a control instruction transmitted by the console through the remote communication system, parsing the control instruction so as to determine an end pose of the surgical robot arm, and transmitting own status information through the remote communication system; and the console is used for generating the control instruction, transmitting the control instruction through the remote communication system, and receiving the status information transmitted by the surgical robot arm through the remote communication system.

Specifically, in the present embodiment, the remote surgery system based on the laparoscopic surgery robot includes the laparoscopic surgery robot system and the remote communication system, wherein the laparoscopic surgery robot system includes the surgical robot arm and the console; the surgical robot arm is located on the patient side and used for performing surgery on a patient; and the console is located on the doctor side, and a doctor controls the surgical robot arm and surgical instruments to perform the laparoscopic minimally invasive surgery on the patient through a main manipulator on the console.

When the patient side and the doctor side are not in a same physical space, data needs to be transmitted between the surgical robot arm and the console through the remote communication system, and the data includes the control instruction and the status information, wherein the control instruction is generated when the doctor operates the main manipulator of the console, and the control instruction is transmitted to the surgical robot arm through the remote communication system; the surgical robot arm performs the parsing and calculation according to the kinematics algorithm to determine the end pose; joints of the main manipulator and the end pose are matched one by one, that is, the end pose refers to a position and posture status of an end point of the surgical instrument; the status information refers to a status of the surgical robot arm; and the status of the surgical robot arm is fed back to the console, and the doctor is informed of the status of the surgical robot arm through a signal light on the doctor side, so that the doctor adjusts the surgical situation in time.

The control instruction is processed in a single-ended data mode; if the console and the surgical robot arm perform the data calculation simultaneously, due to the time delay of the data transmission, the time delay of devices may be caused when the data such as the end pose determined by the console is transmitted to the surgical robot arm, which may affect the synchronism of the surgery, thereby adopting the single-ended data mode in which the parsing and calculation are performed only at the side of the surgical robot arm, that is, the data calculation is performed by using the algorithm only at the surgical robot arm without the anticipation of the console, so that the time delay of the devices occupied by the console and the surgical robot arm calculating the data separately is reduced, thereby ensuring the real-time performance of surgical operations.

In the present embodiment, the remote surgery on the patient by the doctor is realized through the remote communication system, so that the surgery can be performed when the patient side and the doctor side are not in the same physical space, thereby enabling the wide-spread access to high-quality medical resources; and since the control instructions are subjected to the data calculation by using the algorithm only at the surgical robot arm, the time delay of devices occupied by the console and the surgical robot arm calculating the data separately is reduced, thereby ensuring the real-time performance of the surgical operations.

Optionally, the remote surgery system based on the laparoscopic surgery robot also includes a 3D intra-cavity video processing system and a 3D display located on the doctor side; the 3D intra-cavity video processing system includes a laparoscopic lens, an intra-cavity image collection unit and a video encoding/decoding system; the laparoscopic lens is installed on the surgical robot arm; the laparoscopic lens is used for collecting focus information of the patient; the intra-cavity image collection unit is used for receiving the focus information collected by the laparoscopic lens and transmitting the focus information to the video encoding/decoding system; and the video encoding/decoding system is used for encoding and decoding the focus information and transmitting to the 3D display through the remote communication system.

Specifically, in the present embodiment, as shown in FIG. 2, the remote surgery system based on the laparoscopic surgery robot also includes the 3D intra-cavity video processing system and the 3D display located at the doctor side; the 3D intra-cavity video processing system is used for providing a view for the doctor to perform the remote surgery, so that details of a focus in an abdominal cavity of the patient can be detected clearly; the 3D intra-cavity video processing system includes the laparoscopic lens, the intra-cavity image collection unit and the video encoding/decoding system, wherein the laparoscopic lens is installed on the surgical robot arm and fed into the abdomen of the patient through a minimally invasive hole to acquire the focus information of the patient; the other end of the laparoscopic lens is connected to the intra-cavity image collection unit through a cable; the intra-cavity image collection unit is connected to the video encoding/decoding system through a DVI cable; the focus information is encoded by a video transmission device of the video encoding/decoding system, transmitted to a video receiving device through the remote communication system for decoding and restoration, and then transmitted to the 3D display, so that an intra-cavity image, i.e. the focus information is displayed at the doctor side, and the doctor adjusts the surgical situation in time.

In the present embodiment, the visualization of a surgical procedure is realized through the 3D intra-cavity video processing system, so that the doctor adjusts the surgical situation in time, thereby effectively improving the safety of the laparoscopic surgery.

Optionally, the video encoding/decoding system includes a video capture card, a video transmission device and a video receiving device; the video capture card is connected with the intra-cavity image collection unit; the video capture card is used for receiving the focus information transmitted by the intra-cavity image collection unit and transmitting the focus information to the video transmission device; the video transmission device is used for encoding the focus information and transmitting to the video receiving device through the remote communication system; and the video receiving device is used for receiving the focus information transmitted by the video transmission device, decoding the focus information and transmitting the decoded focus information to the 3D display, so that the focus information is displayed on the 3D display.

Specifically, in the present embodiment, as shown in FIG. 2, the video encoding/decoding system includes the video capture card, the video transmission device and the video receiving device, wherein the intra-cavity image collection unit is connected with the video capture card through the DVI cable; the video capture card is connected with the video transmission device through a dedicated connection line; and the video transmission device encodes a 3D laparoscopic image containing the focus information through an encoding technology and transmits data of the 3D laparoscopic image to the video receiving device through a UDP protocol for decoding, so that the focus information is displayed on the 3D display.

In the present embodiment, the 3D laparoscopic image containing the focus information is encoded/decoded by the video encoding/decoding system to realize the visualization of the surgical procedure, so that the doctor adjusts the surgical situation in time, thereby effectively improving the safety of the laparoscopic surgery.

Optionally, the remote communication system includes a video transmission communication system, a video receiving communication system and a network base station; the video transmission communication system is connected with the video transmission device; the video transmission communication system is used for receiving the focus information transmitted by the video transmission device and transmitting the focus information to the video receiving communication system through the network base station; the network base station is communicated and connected with the video transmission communication system and the video receiving communication system; the network base station is used for receiving the focus information transmitted by the video transmission communication system and transmitting the focus information to the video receiving communication system; the video receiving communication system is connected with the 3D display; the video receiving communication system is used for receiving the focus information transmitted by the network base station and transmitting the focus information to the 3D display; and the video transmission communication system and the video receiving communication system are suitable for transmitting the focus information through a first dedicated circuit.

Specifically, in the present embodiment, as shown in FIG. 2, the remote communication system includes the video transmission communication system, the video receiving communication system and the network base station, wherein the video transmission device is connected with the video transmission communication system through a network cable; the video transmission communication system is communicated with the video receiving communication system through the network base station so as to realize the data communication; the video receiving communication system is connected with the video receiving device through a network cable; and the video receiving device is connected with the 3D display on the doctor side through a DVI connection line, so that the intra-cavity image is displayed on the 3D display.

The focus information is transmitted between the video transmission communication system and the video receiving communication system through the first dedicated circuit, which does not interfere with other data transmission, so that the mutual interference is reduced, the collateral damage caused by the network instability is prevented, and the reliability of the data transmission is ensured.

In the present embodiment, the 3D laparoscopic image containing the focus information is transmitted by the remote communication system to realize the visualization of the surgical procedure, so that the doctor adjusts the surgical situation in time, thereby effectively improving the safety of the laparoscopic surgery; and the same time, since the focus information is transmitted between the video transmission communication system and the video receiving communication system through the first dedicated circuit, which does not interfere with other data transmission, the mutual interference is reduced, the collateral damage caused by the network instability is prevented, and the reliability of the data transmission is ensured.

Optionally, the video transmission device is suitable for adopting the UDP protocol when transmitting the focus information to the video receiving device.

Specifically, in the present embodiment, the video transmission device is suitable for adopting the UDP protocol when transmitting the focus information to the video receiving device, which ensures relatively high real-time performance of the data transmission, and gives the doctor good surgical experience, so that the doctor adjusts the surgical situation in time, thereby effectively improving the safety of the laparoscopic surgery.

In the present embodiment, through the UDP protocol, the focus information is transmitted by the video transmission device to the video receiving device, which ensures the relatively high real-time performance of the data transmission, and gives the doctor good surgical experience, so that the doctor adjusts the surgical situation in time, thereby effectively improving the safety of the laparoscopic surgery.

Optionally, the video transmission device is suitable for adopting a low-distortion encoding algorithm when encoding the focus information.

Specifically, in the present embodiment, the video transmission device is suitable for adopting the low-distortion encoding algorithm when encoding the focus information; since the laparoscopic image on the patient side is a 1080P high-definition 3D image which mainly reflects the situation of an intra-cavity focus of the patient, the remote transmission of the image shall ensure relatively high definition and continuity, so that the doctor can acquire accurate situation of the patient for performing the surgery. Therefore, the high-definition 1080P video capture card is used for local collection; the low-distortion encoding algorithm is performed at the video transmission device; and when a network status is affected, a compression ratio of the 3D image video can be adjusted according to the network status, which ensures relatively high continuity of the 3D image.

In the present embodiment, the focus information is encoded through the low-distortion encoding algorithm; when the network status is affected, the compression ratio of the 3D image video may be adjusted according to the network status, which ensures the high continuity of the 3D image, so that the doctor adjusts the surgical situation in time, thereby effectively improving the safety of the laparoscopic surgery.

Optionally, the remote communication system also includes a robot arm communication system and a console communication system; the robot arm communication system is connected with the surgical robot arm; the console communication system is connected with the console; the robot arm communication system is communicated and connected with the console communication system; and the robot arm communication system and the console communication system are suitable for transmitting the control instruction and the status information through a second dedicated circuit.

Specifically, in the present embodiment, as shown in FIG. 2, the remote surgery system based on the laparoscopic surgery robot also includes the robot arm communication system and the console communication system; the robot arm communication system is connected with the surgical robot arm; the console communication system is connected with the console; and the robot arm communication system is communicated and connected with the console communication system. The data transmission between the surgical robot arm and the console communication system is realized through the robot arm communication system and the console communication system, which enables the remote surgery on the patient by the doctor, so that the surgery can be performed when the patient side and the doctor side are not in the same physical space, thereby enabling the wide-spread access to the high-quality medical resources.

The control instruction and the status information are transmitted between the robot arm communication system and the console communication system through the second dedicated circuit, which does not interfere with other data transmission, so that the mutual interference is reduced, the collateral damage caused by the network instability, and the reliability of the data transmission is ensured.

In order to ensure the safety of the remote surgery, the control instruction and the 3D intra-cavity image are transmitted respectively by respective dedicated network transmission channels; and the dedicated network transmission channels refer to two point-to-point fixed IPs and transmit the data correspondingly one by one, so that the mutual interference is reduced, the collateral damage caused by the network instability is reduced, and the reliability of the data transmission is ensured.

In the present embodiment, the data transmission between the surgical robot arm and the console communication system is realized through the robot arm communication system and the console communication system to enable the remote surgery on the patient by the doctor, so that the surgery can be performed when the patient side and the doctor side are not in the same physical space, thereby enabling the wide-spread access to the high-quality medical resources; and at the same time, since the control instruction and the status information are transmitted between the robot arm communication system and the console communication system through the second dedicated circuit, which does not interfere with other data transmission, the mutual interference is reduced, the collateral damage caused by the network instability is prevented, and the reliability of the data transmission is ensured.

Optionally, the surgical robot arm determines the end pose based on a kinematics algorithm.

Specifically, in the present embodiment, the surgical robot arm determines the end pose based on the kinematics algorithm to realize the remote surgery on the patient by the doctor, so that the surgery can be performed when the patient side and the doctor side are not in the same physical space, thereby enabling the wide-spread access to the high-quality medical resources.

In the present embodiment, the end pose of the surgical robot arm is determined through the kinematics algorithm to enable the remote surgery on the patient by the doctor, so that the surgery can be performed when the patient side and the doctor side are not in the same physical space, thereby enabling wide-spread access to the high-quality medical resources.

Optionally, the surgical robot arm includes a surgical robot arm body and a surgical instrument apparatus for performing surgery on the patient; and the surgical instrument apparatus is installed on the surgical robot arm body.

Specifically, in the present embodiment, as shown in FIG. 2, the remote surgery system based on the laparoscopic surgery robot also includes the surgical robot arm body and the surgical instrument apparatus; and the surgical instrument apparatus is installed on the surgical robot arm body and used for moving together with the surgical robot arm body and adjusting own status according to the end pose determined by the control instruction.

In the present embodiment, through the surgical instrument apparatus installed on the surgical robot arm, the remote surgery on the patient by the doctor is enabled, so that the surgery can be performed when the patient side and the doctor side are not in the same physical space, thereby enabling wide-spread access to the high-quality medical resources.

Another embodiment of the present invention provides a control method based on a laparoscopic surgery robot, which includes: a control instruction is generated and the control instruction is transmitted to a surgical robot arm through a remote communication system by a console; the control instruction is parsed by the surgical robot arm so as to determine an end pose of the surgical robot arm; and status information of the surgical robot arm is transmitted to the console by the surgical robot arm.

A remote surgical procedure is taken as an example:

For example, the remote surgery from Beijing to Suzhou is performed; the patient side is located in Suzhou, and the doctor side is located in Beijing; surgical scenarios on both sides are arranged respectively; the robot arm system (including the surgical robot arm and the surgical instrument apparatus), the laparoscopic lens, the video capture card, the video transmission device, the robot arm communication system and the video transmission communication system on the patient side are connected and powered on respectively; and at the same time, the console, the video receiving communication system, the video receiving device, the 3D display and the console communication system on the doctor side are connected and powered on respectively. After the devices on both the doctor side in Beijing and the patient side in Suzhou are powered on and operated normally, the robot arm system on the patient side examines various modules and resets the robot arm. After the robot arm system is reset, the robot arm system, the doctor console, the video transmission device and the video receiving device input a configured IP address respectively for UDP data transmission and video transmission; and at the time, the doctor side can see the situation of the remote patient through the 3D display, and the doctor console is remotely connected with the robot arm system, ready for the surgery. The doctor is assisted for perforation according to a position of a focus, and the active positioning of the robot arm is performed; after the surgical instrument is installed, the doctor can control the main manipulator of the doctor console to remotely control the surgical instrument to perform the surgery on the patient; in the surgical procedure, the operation of the doctor through the main manipulator may generate the corresponding control instruction which is transmitted to the surgical robot arm through the remote communication system, and the control instruction is parsed at the surgical robot arm side to determine the end pose, so that the position and posture of the surgical robot arm and the surgical instrument apparatus are adjusted to completely match the end pose of the robot arm instrument with the pose of the main manipulator, thereby enabling the master-slave follow-up movement to complete the surgery on the patient; since the data calculation is performed simultaneously at the console and the surgical robot arm, and the data transmission has time delay, which may result in the time delay of the devices when the data such as the end pose determined by the console is transmitted to the surgical robot arm, and may affect the synchronism of the surgery, the single-ended data mode in which the parsing and calculation are performed only at the surgical robot arm side is adopted to effectively ensure the synchronism of the remote surgery; and after the surgery is completed, the doctor needs to arrange the main manipulator on a reset position, so that the machine is restored to an initial standby state.

Although the present invention is disclosed as above, the protection scope of the present invention is not limited thereto. Various changes and modifications may be performed by those skilled in the art without departing from the spirit and scope of the present invention, and these changes and modifications fall within the protection scope of the present invention.

## Claims

1. A remote surgery system based on a laparoscopic surgery robot, **characterized by** comprising a laparoscopic surgery robot system and a remote communication system, wherein the laparoscopic surgery robot system comprises a surgical robot arm located on a patient side and a console located on a doctor side; the surgical robot arm and the console are suitable for communication connection by means of the remote communication system;
the surgical robot arm is used for receiving a control instruction transmitted by the console through the remote communication system, parsing the control instruction so as to determine an end pose of the surgical robot arm, and transmitting own status information through the remote communication system; and the console is used for generating the control instruction, transmitting the control instruction through the remote communication system, and receiving the status information transmitted by the surgical robot arm through the remote communication system.

2. The remote surgery system based on the laparoscopic surgery robot according to claim 1, **characterized by** further comprising a 3D intra-cavity video processing system and a 3D display located on the doctor side, wherein the 3D intra-cavity video processing system comprises a laparoscopic lens, an intra-cavity image collection unit and a video encoding/decoding system;
the laparoscopic lens is installed on the surgical robot arm; and the laparoscopic lens is used for collecting focus information of the patient;
the intra-cavity image collection unit is used for receiving the focus information collected by the laparoscopic lens and transmitting the focus information to the video encoding/decoding system; and
the video encoding/decoding system is used for encoding/decoding the focus information and transmitting to the 3D display through the remote communication system.

3. The remote surgery system based on the laparoscopic surgery robot according to claim 2, **characterized in that** the video encoding/decoding system comprises a video capture card, a video transmission device and a video receiving device;
the video capture card is connected with the intra-cavity image collection unit; the video capture card is used for receiving the focus information transmitted by the intra-cavity image collection unit and transmitting the focus information to the video transmission device;
the video transmission device is used for encoding the focus information and transmitting to the video receiving device through the remote communication system; and
the video receiving device is used for receiving the focus information transmitted by the video transmission device, decoding the focus information and transmitting the decoded focus information to the 3D display, so that the focus information is displayed on the 3D display.

4. The remote surgery system based on the laparoscopic surgery robot according to claim 3, **characterized in that** the remote communication system comprises a video transmission communication system, a video receiving communication system and a network base station;
the video transmission communication system is connected with the video transmission device; the video transmission communication system is used for receiving the focus information transmitted by the video transmission device and transmitting the focus information to the video receiving communication system through the network base station;
the network base station is communicated with the video transmission communication system and connected with the video receiving communication system; the network base station is used for receiving the focus information transmitted by the video transmission communication system and transmitting the focus information to the video receiving communication system;
the video receiving communication system is connected with the 3D display; the video receiving communication system is used for receiving the focus information transmitted by the network base station and transmitting the focus information to the 3D display; and
the video transmission communication system and the video receiving communication system are suitable for transmitting the focus information through a first dedicated circuit.

5. The remote surgery system based on the laparoscopic surgery robot according to claim 3, **characterized in that** the video transmission device is suitable for adopting a UDP protocol when transmitting the focus information to the video receiving device.

6. The remote surgery system based on the laparoscopic surgery robot according to claim 3, **characterized in that** the video transmission device is suitable for adopting a low-distortion encoding algorithm when encoding the focus information.

7. The remote surgery system based on the laparoscopic surgery robot according to claim 4, **characterized in that** the remote communication system also comprises a robot arm communication system and a console communication system; the robot arm communication system is connected with the surgical robot arm; the console communication system is connected with the console; and the robot arm communication system is communicated and connected with the console communication system; and
the robot arm communication system and the console communication system are suitable for transmitting the control instructions and the status information through a second dedicated circuit.

8. The remote surgery system based on the laparoscopic surgery robot according to claim 1, **characterized in that** the surgical robot arm determines the end pose based on a kinematics algorithm.

9. The remote surgery system based on the laparoscopic surgery robot according to any one of claims 1-8, **characterized in that** the surgical robot arm comprises a surgical robot arm body and a surgical instrument apparatus for performing the surgery on the patient; and the surgical instrument apparatus is installed on the surgical robot arm body.

10. A control method based on a laparoscopic surgery robot, **characterized by** comprising:
generating a control instruction and transmitting the control instruction to a surgical robot arm through a remote communication system by a console;
parsing, by the surgical robot arm, the control instruction so as to determine an end pose of the surgical robot arm; and
transmitting, by the surgical robot arm, status information of the surgical robot arm to the console.
